# EUROPEAN PATENT SPECIFICATION

(11) **EP 1 723 168 B1**
(45) Date of publication and mention of the grant of the patent: **07.04.2010**
(21) Application number: 05708546.6
(22) Date of filing: 17.02.2005
(51) Int. Cl.: C07K 14/005, C12N 7/04, C12N 15/11

(54) **BEAK AND FEATHER DISEASE VIRUS SEQUENCES, COMPOSITIONS AND VACCINES AND THE USE THEREOF IN THERAPY, DIAGNOSIS AND ASSAYS**
SEQUENZEN DES SCHNABEL- UND FEDERKRANKHEIT-VIRUS, ZUSAMMENSETZUNGEN UND IMPFSTOFFE UND DEREN VERWENDUNG IN DER THERAPIE, DER DIAGNOSE UND IN ASSAYS
SEQUENCES DU VIRUS DE LA MALADIE DU BEC ET DES PLUMES, COMPOSITIONS ET VACCINS ASSOCIES, ET LEUR UTILISATION DANS DES METHODES THERAPEUTIQUES ET DIAGNOSTIQUES ET DANS DES DOSAGES

(30) Priority: 17.02.2004 ZA 200401266
(43) Date of publication of application: 22.11.2006
(60) Divisional application: 09154260.5
(73) Proprietor: University of Cape Town, Rondebosch 7701 (ZA)
(72) Inventor: RYBICKI, Edward Peter, 7405 Cape Town (ZA); WILLIAMSON, Anna-Lise, 7405 Cape Town (ZA); HEATH, Livio, 7780 Western Cape (ZA)
(74) Representative: Brand, Thomas Louis
(86) International application number: PCT/IB2005/000405
(87) International publication number: WO 2005/082929

(56) References cited:
- WO-A-99/45956
- BASSAMI M R ET AL: "Genetic diversity of beak and feather disease virus detected in psittacine species in Australia." VIROLOGY. 20 JAN 2001, vol. 279, no. 2, 20 January 2001 (2001-01-20), pages 392-400, XP002349035 ISSN: 0042-6822 cited in the application
- NOTEBORN M H M ET AL: "SIMULTANEOUS EXPRESSION OF RECOMBINANT BACULOVIRUS-ENCODED CHICKEN ANAEMIA VIRUS (CAV) PROTEINS VP1 AND VP2 IS REQUIRED FOR FORMATION OF THE CAV-SPECIFIC NEUTRALIZING EPITOPE" JOURNAL OF GENERAL VIROLOGY, SOCIETY FOR GENERAL MICROBIOLOGY, READING, GB, vol. 79, 1998, pages 7073-7077, XP000904640 ISSN: 0022-1317
- KIM YUNA ET AL: "Characterization of the recombinant proteins of porcine circovirus type2 field isolate expressed in the baculovirus system." JOURNAL OF VETERINARY SCIENCE (SUWON-SI, KOREA) MAR 2002, vol. 3, no. 1, March 2002 (2002-03), pages 19-23, XP002349036 ISSN: 1229-845X

## Description

### BACKGROUND OF THE INVENTION

The invention relates to isolated Beak and feather disease virus (BFDV) sequences and to the use of BFDV proteins and nucleic acid sequences for preparing pharmaceutical compositions intended to generate an immunological response against BFDV. The invention further relates to compositions for prophylactic treatment of beak and feather disease (BFD) and diagnostic kits for use in the diagnosis of BFDV infections.

In this specification the following terms, phrases and/or clauses are to be understood to mean:
Construct - as used herein is synonymous with terms such as conjugate, cassette and hybrid, and includes the nucleotide sequence according to the invention, or parts thereof, directly or indirectly linked to a promoter. The construct may contain or express a marker, which allows for the selection for the construct in a host cell.
Expression vector - as used herein a construct capable of *in vivo* or *in vitro* expression.
Expression is understood to mean the production of a protein from a DNA template via transcription and translation.
Fragment of BFDV protein - as used herein is intended to denote, in particular, any fragment of an amino acid sequence of a BFDV protein which is capable of generating an immunogenic response directed against BFDV.
Nucleotide sequence - as used herein is synonymous with the term nucleotide acid sequence and/or the term polynucleotide, and includes genomic DNA, cDNA, recombinant DNA and RNA, and any combination of the aforementioned. The nucleotide sequence may be double-stranded or single-stranded, whether representing the sense or the antisense strand. Typically, the term nucleotide sequence means DNA.
Homologue - as used herein means an entity having a certain homology with amino acid sequences or nucleotide sequences.
Protein - as used herein is intended to denote both peptides and polypeptides.
TMV - as used herein indicates Tobacco mosaic virus.
Vector - as used herein includes expression vectors, replication vectors, transformation vectors, shuttle vectors, cosmids, plasmids, phages, viruses and yeast artificial chromosomes or any combination thereof.

A growing number of avian diseases have recently been attributed to circoviruses or circovirus-like infections (Todd, 2000; Woods & Latimer, 2000). These viruses are characterised by small single-stranded circular DNA genomes encapsulated by non-enveloped spherical capsids (Ritchie *et al.,* 1989). Currently, the taxonomic family *Circoviridae* includes *Chicken anaemia virus* (Notebom *et al.,* 1991), the only member of the genus *Gyrovirus; Porcine circovirus* 1 and 2 (Meehan *et al.,* 1997; Meehan *et al.,* 1998); and *Beak and feather disease virus* (Bassami *et al.,* 1998; Niagro *et al.,* 1998), all of which constitute the genus Circovirus. The general genome structure and organisation of the tentative members of the circovirus family, Colimbid circovirus, Pigeon circovirus, Duck circovirus, Canary circovirus and Goose circovirus, suggest that these viruses should also be assigned to the *Circovirus* genus (Phenix *et al.,* 2001).

Psittacine beak and feather disease (PBFD) was first describe in 1975 by Dr Ross Perry and has since been recognised as the most significant infectious disease in psittacine birds (Pass & Perry, 1984; Raidal *et al.,* 1993). The disease is characterised by feather dystrophy and loss, sometimes coupled with deformities of the beak and claws. Circovirus infections are commonly associated with immunodeficiency-related diseases caused be the depletion of lymphoid tissue (Ritchie et al., 1989). In the case of beak and feather disease, damage to the lymphoreticular tissue is most pronounced in the brusa of Fabricius and the thymus (Latimer *et al.,* 1991). Affected birds frequently succumb to secondary infections.

The virus can be transmitted horizontally between birds as well as vertically via the egg (Ritchie & Carter, 1995). Infections are usually acquired by ingestion and inhalation of contaminated feather dust. It is also possible to establish acute viral infections by introducing infectious material via intracloacal, intranasal, subcutaneous and intermuscular routes (Ritchie & Carter, 1995). Experimental data suggest that the severity of the disease is dependent on the dose and route of infection as well as the species involved (Wylie & Pass, 1987; Raidal *et al.,* 1993). The disease has been reported in many species of captive and wild Old World psittacine birds (Pass & Perry, 1984; McOrist, 1989). Viral infections have also been documented in New World parrots, but clinical observations suggest that these psittaforms are less susceptible to the virus than Old World species (Huff *et al*., 1988; Ritchie *et al*., 1990; Greenacre *et al*., 1992). The virus is endemic in Australia, but has spread to Asia, Europe, North and South America and Africa (Bassami *et al.,* 2001). The disease prevalence in flocks of wild cockatoos in Australia is estimated to be as high as 20%, with seroprevalence of between 60 and 80% (Raidal *et al.,* 1993). The disease poses a serious threat to the survival of several psittacine species, including the highly endangered Cape parrot and Black-cheek lovebird.

There is no cure for birds affected by PBFD and birds with chronic infections do not recover from the disease. Birds with active BFDV infections have been found to have significantly lower antibody titres compared to birds that have exposed to virus but remain clinically normal (Ritchie *et al.,* 1992), suggesting that some birds are able to mount an immune response effectively controlling the infection and thereby reducing the severity of the disease. With this in mind, an inactivated vaccine has been developed, which is able to stimulate immunity to the virus (Ritchie *et al.,* 1992; Raidal *et al.,* 1993). This vaccine, however, confers only partial protection against challenge with purified BFDV.

A significant drawback of inactivated vaccines is the threat of residual infectivity. In one challenge study, three sibling wild-caught sulphur-crested cockatoos died shortly after being vaccinated. This was probably due to improperly inactivated vaccine preparations (Raidal *et al.,* 1993). This problem can, however, be circumvented by the production of subunit vaccines. Subunit vaccines consist of only part of the virus and thus pose no threat of introducing infectious material during vaccination.

The genome of PBFD contains two open reading frames, which occur on the virus and complementary-sense strands and encode the replication-associated and capsid proteins respectively (Niagro *et al.,* 1998). The coat protein is the major constituent of the infectious viral particle and is the likely target of the immune system. The deduced amino acid sequence of the coat protein of several BFDV isolates has been shown to differ by as much as 27% (Bassami *et al*., 2001). However, these isolates appear to be antigenically similar in haemagglutination and haemagglutination inhibition assays (Ritchie et al., 1990).

The captive breeding of psittacines is a highly lucrative and growing market. The breeding and export of psittacine birds are severely hampered by the disease and more than R24 million in revenue is lost annually due to the disease. The development of a safe and effective vaccine would be invaluable to breeders.

In the continued absence of an effective vaccine, disease surveillance in both wild and captive populations remains central to the management of BFD. Currently, the haemagglutination and haemagglutination inhibition assays are the preferred methods for detecting the virus in affected feathers and antibodies in blood, serum, plasma or yolk, respectively (Raidal *et al.,* 1993; Ritchi *et al.,* 1991; Sanada *et al*., 2000). In addition, a universal PCR assay has been developed that consistently detected the virus in psittacine birds. The assay uses primers within open reading frame 1 (ORF1) of the BFDV genome (Ypelaar *et al.,* 1999) and enables one to identify birds that are clinically infected with the disease as well as those that are latently infected, i.e. carrying the virus but displaying no clinical symptoms. The PCR test is currently the preferred method of diagnosis in suspected cases of PFBD, and has also been used routinely in South Africa. However, none of the above mentioned methods would be able to distinguish between birds that have been vaccinated and those that are infected with the PBFD virus. There is therefore also a need for a commercial BFDV antibody detection assay for use in determining whether or not birds have been vaccinated, and to detect past BFDV infections.

### SUMMARY OF THE INVENTION

According to a first embodiment of the invention, there is provided a composition comprising a beak and feather disease virus (BFDV) coat protein polypeptide:
(a) having an amino acid sequence which has at least 80% identity to a sequence set out in any one of SEQ ID NOS: 5 to 8 which is capable of generating an immunogenic response directed against a circovirus and wherein in said composition said protein encapsidates a circovirus-derived single stranded circular nucleotide sequence containing replication origin and cis-acting sequences necessary for replication, wherein:
(b) the encapsidated single-stranded circular nucleotide sequence is replicated by BFDV Rep expressed in trans;
(c) the encapsidated single-stranded circular nucleotide sequence is a native BFDV genome, and constitutes an infectious virion when encapsidated by the BFDV coat protein;
(d) the encapsidated single-stranded circular nucleotide sequence is a BFDV genome with its Rep gene replaced, and is non-replicating in transfected cells or in birds; or
(e) the encapsidated single-stranded circular nucleotide sequence is a BFDV genome with its coat protein gene replaced with another gene, and is replication-competent in transfected cells or in birds;
   for use in a method of preventing and/or treating a circovirus infection.

The nucleotide sequence may be:
a. a homologue, variant and/or derivative of the nucleotide sequence described above;
b. a complement of the nucleotide sequence; or
c. a complement of the homologue, variant and/or derivative of (a).

The circovirus may be a Beak and Feather disease virus or a related virus, such as Pigeon circovirus, Duck circovirus, Goose circovirus or Columbid circovirus..

Also described is a gene construct including a nucleotide sequence described above and a promoter.

Also described is a vector including and/or expressing:
a. an isolated nucleotide sequence set out in any one of SEQ ID NOS: 1 to 4, or fragment thereof;
b. a homologue, variant and/or derivative of the nucleotide sequences set out in any one of SEQ ID NOS: 1 to 4, or fragment thereof;
c. a complement of the isolated nucleotide sequences set out in any one of SEQ ID NOS: 1 to 4, or fragment thereof;
d. a complement of the isolated homologue, variant, and/or derivative or the nucleotide sequences set out in any one of SEQ ID NOS: 1 to 4, or fragment thereof;
e. a nucleotide sequence that is capable of hybridising to:
   i. any one of the isolated nucleotide sequences, or fragment thereof,
   ii. a homologue, variant and/or derivative of the nucleotide sequence, or fragment thereof,
   iii. a complement of the isolated nucleotide sequences, or fragment thereof,
   iv. a complement of the isolated homologue, variant and/or derivative of any one of the nucleotide sequences, or fragment thereof; or
   v. any combination of (e)(i)-(e)(iv); or
f. any combination of (a)-(e);
   and a promoter which is operably linked to the sequence of any one of (a)-(f) or any combination of (a)-(f).

Also described is a method of producing a Beak and feather disease virus (BFDV) protein, or fragment thereof, the method including the steps of:
a. contacting a bacterial cell and/or insect cell via baculovirus and/or plant cell with a vector and/or construct as described herein; and
b. cultivating the bacterial cell, insect cell and/or plant cell under conditions suitable for the production of the BFDV protein or part thereof.

The bacterial cell may be *Escherichia coli*, the insect cell may be a *Spodoptera frugiperda* cell and the plant cell may be a *Nicotiana benthamiana* cell.

The protein has at least 80%, preferably 90% and even more preferably at least 95% identity to any one of the amino acid sequences set out in SEQ ID NOS: 5 to 8. Also described is the use of a beak and feather disease virus (BFDV) protein, or fragment thereof, which includes:
a. the amino acid sequence derived from the nucleotide sequence set out in any one of SEQ ID Nos: 1 to 4;
b. the amino acid sequence of a nucleotide sequence having at least 70%, preferably 80% and more preferably 90% homology, after optimal alignment, with the nucleotide sequence set out in any one of SEQ ID Nos: 1 to 4;
c. an amino acid sequence of a fragment of a BFDV protein as defined in (a); or
d. an amino acid sequence set out in any one of SEQ ID NOS: 5 to 8, or which has at least 80%, preferably 90% and even more preferably at least 95% identity to these sequences;

in the manufacture of a pharmaceutical composition for use in a method of treatment or prophylaxis of a circovirus.

Also described is the use of a cell culture system expressing BFDV coat protein (CP) to encapsidate circovirus-derived single-stranded circular nucleotide sequence containing replication origin and cis-acting sequences necessary for replication, where:
a. the nucleotide sequence is replicated in the cell culture by expression either in *trans* (from another construct) or in *cis* (from the same construct) of BFDV Rep;
b. the nucleotide sequence is a native BFDV genome, and constitutes infectious virions when encapsidated by BFDV CP;
c. the nucleotide sequence constitutes a BFDV genome with the Rep gene replaced, and is non-replicating in transfected cells or in birds; or
d. the nucleotide sequence constitutes a BFDV genome with the CP gene replaced with another gene, and is replication-competent in transfected cells or in birds;
in the manufacture of a pharmaceutical composition for use in a method of treatment or prophylaxis of a circovirus infection.

The cell culture system may be recombinant baculovirus expression in insect cells, or use of stably transfected insect or other animal or plant cells.

The circovirus may be BFDV or relatives thereof, such as Duck circovirus, Goose circovirus, Columbid circovirus, or Pigeon circovirus.

Replacement genes may be marker genes, such as a green fluorescent protein (GFP) gene, or genes coding for proteins of interest for use as vaccines in psittacines or other animals.

Also described is a method of detecting Beak and feather disease virus (BFDV) infection in a candidate suspected of BFDV infection, the method including the steps of:
a. contacting a suitable sample of the candidate's tissue with a protein or fragment thereof which is coded by a nucleotide sequence described above; and
b. detecting a successful binding event between an antibody and the protein or protein fragment.

Also described is a method of detecting a circovirus infection, such as Beak and feather disease virus (BFDV) infection, in a candidate suspected of the infection, the method including the steps of:
a. contacting a suitable sample of the candidate's tissue with a probe containing the protein or protein fragment described above; and;
b. detecting a successful binding event between the probe and the protein or protein fragment.

The binding event may be detected by detection of a marker associated with the probe. The marker may be selected from the group consisting of fluorescent markers, radioisotope markers, colorimetric markers or the like.

Also described is a diagnostic kit for use in the diagnosis of conditions associated with circovirus infections, such as Beak and feather disease virus infection, which includes the protein or fragment of a protein and the probe, both described above.

According to a further embodiment of the invention, there is provided an assay for quantification of serum antibody immune response in vaccinated birds, which includes the compositions in accordance with the invention, or infectious virions formed thereby and their use in cell cultures or in whole animals.

Also described is a composition including a protein or protein fragment encoded by any one of the nucleotide sequences described above, or an antibody that has been illicited in response to the protein or protein fragment. The composition may be used for treating, diagnosing or assaying a circovirus infection.

Also described is an isolated antibody for preventing and/or treating a circovirus infection, which is prepared by:
(a) immunizing a laying hen with a protein or fragment thereof encoded by a nucleotide sequence as described herein; and
(b) isolating the antibody from eggs of the hen produced in response to the protein or fragment.

The invention also provides a recombinant vaccine for BFDV comprising a pseudovirion comprising a BFDV polypeptide having an amino acid sequence set out in any one of SEQ ID NOS: 5 to 8 produced from a DNA construct in cell culture encapsidating single-stranded circular (ssc) virus-derived recombinant nucleotide sequence made in the same cells by the agency of the viral Rep (replication-associated protein), express in cis or in trans.

### BRIEF DESCRIPTION OF THE DRAWINGS

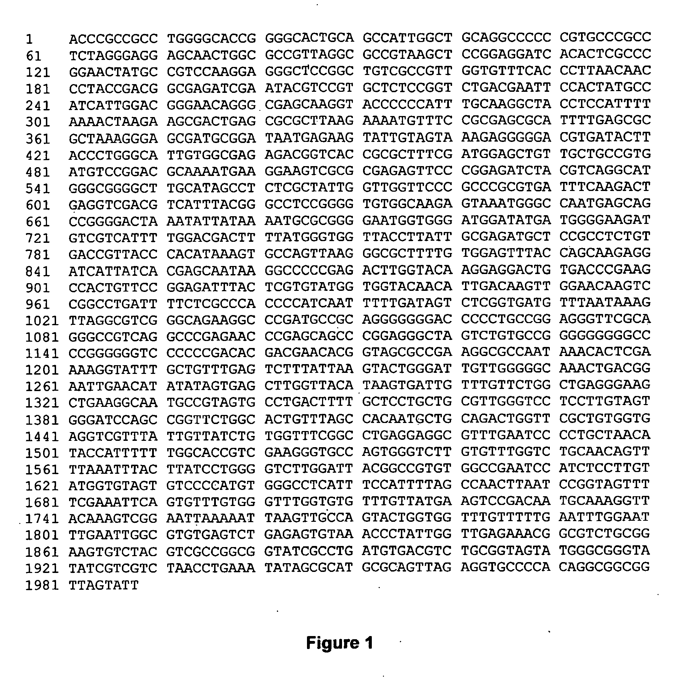

- Figure 1: shows a full-length Beak and feather disease virus (BFDV) genome nucleotide sequence (SEQ ID NO 1) of a first isolate, BFDV AfrG3-RSA;

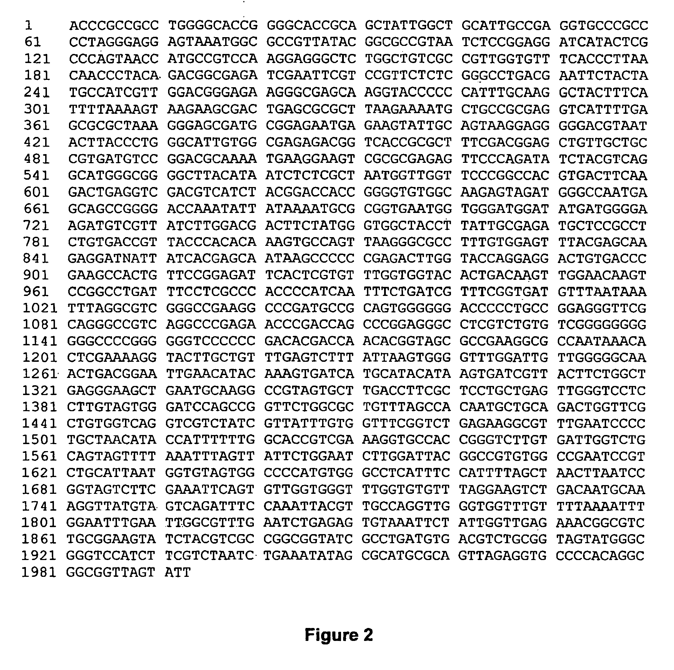

- Figure 2: shows a full-length BFDV genome-nucleotide sequence (SEQ ID NO 2) of a second isolate, BFDV AfrG4-Gp;

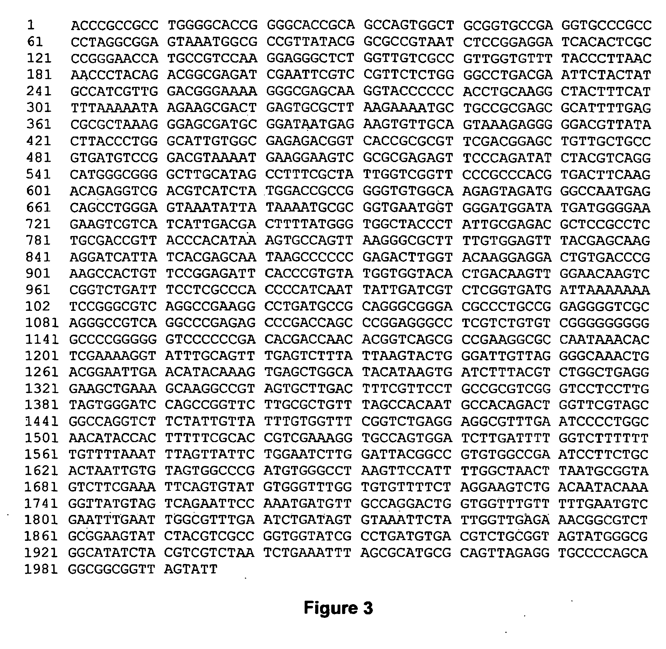

- Figure 3: shows a full-length BFDV genome nucleotide sequence (SEQ ID NO 3) of a third isolate, BFDV UC2-WC;

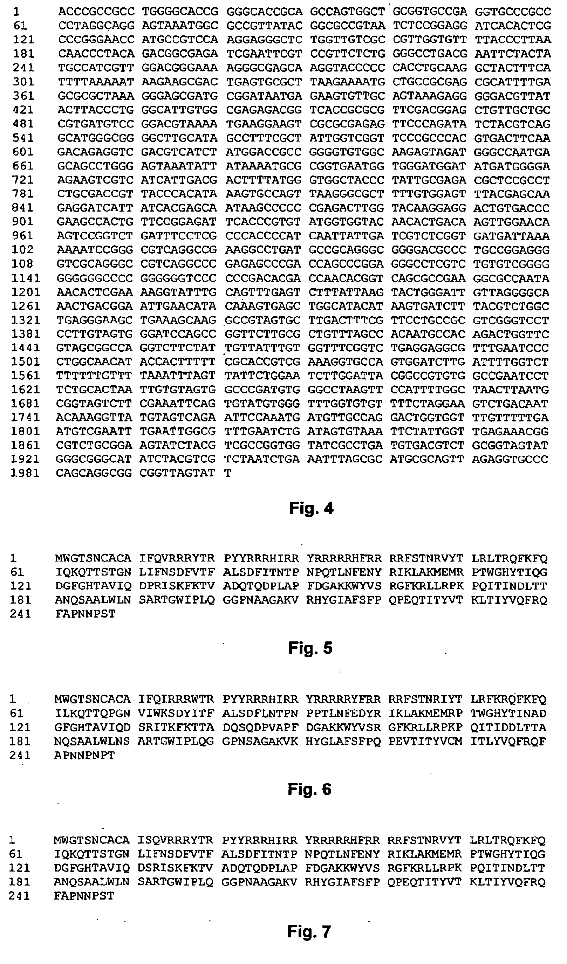

- Figure 4: shows a full-length BFDV genome nucleotide sequence (SEQ ID NO 4) of a fourth isolate, BCL1-ZAM;
- Figure 5 -8: show the BFDV coat protein sequences (SEQ ID NOS: 5-8) of isolates BFDV AfrG3-RSA (corresponds to nt 931-1677 in SEQ ID NO: 1), BFDV AfrG4-Gp (corresponds to nt 1220-1972 in SEQ ID NO: 2), BFDV UC2-WC (corresponds to nt 1229-1979 in SEQ ID NO: 3) and BCL1-ZAM (corresponds to nt 1235-1984 in SEQ ID NO: 4), respectively;
- Figure 9: shows the BFDV coat protein (CP) expressed in *Escherichia coli* detected by Western blot analysis;
- Figure 10: shows the BFDV CP expressed in *Spodoptera frugiperda* cells (Sf21) detected by Western blot analysis. Lysates of uninfected SF21 cells (lane 1), SF21 cells infected with wild-type baculovirus (lane 2), and SF21 infected with recombinant baculovirus expressing the BFDV coat protein;
- Figure 11: shows evidence of the BFDV genomic DNA replicating in *Spodoptera frugiperda* cells (Sf21) by means of polymerase chain reaction analysis. DNA extracts of cells transfected with the BFDV genome monomer (lanes 1 and 4), partial dimer (lanes 2 and 5), and full dimer (lanes 3 and 6). Only the partially dimeric construct released replicating BFDV genomic DNA;
- Figure 12: shows the humeral response in chickens immunized three times with purified BFDV CP expressed by recombinant baculovirus in insect cells (the arrows indicate when the antigen was administered);
- Figure 13: shows a full-length BFDV genome nucleotide sequence (SEQ ID NO: 18) of isolate BCL1-ZAM, isolated from blood collected Black-cheek Lovebird in Zambia;
- Figure 14: shows an amino acid sequence (SEQ ID NO: 19) of the capsid protein of AFG3-ZA (corresponds to nt 1238-1984 in full genome sequence);
- Figure 15: shows a reverse complementary nucleotide sequence (SEQ ID NO: 20) of ΔN20 truncated BFDV capsid protein (corresponds to nt 1234-1907 in full genome sequence);
- Figure 16: shows a reverse complementary nucleotide sequence (SEQ ID NO: 21) of ΔN40 truncated BFDV capsid protein (corresponds to nt 1234-1860 in full genome sequence);
- Figure 17: shows a reverse complementary nucleotide sequence (SEQ ID NO: 22) of ΔN50 truncated BFDV capsid protein (corresponds to nt 1234-1824 in full genome sequence);
- Figure 18: shows an amino acid sequence (SEQ ID NO: 23) of ΔN20 truncated BFDV capsid protein;
- Figure 19: shows an amino acid sequence (SEQ ID NO: 24) of ΔN40 truncated BFDV capsid protein;
- Figure 20: shows an amino acid sequence (SEQ ID NO: 25) of ΔN50 truncated BFDV capsid protein; and
- Figure 21: shows SDA-PAGEA analysis of different BFDV capsid protein truncations (Lanes 1 to 7 show crude lysates of SF21 insect cells infected with recombinant baculovirus expressing the respective proteins; lanes 1-8: Uninfected cells, wt CP, AN20 CP, ΔN40 CP, ΔN50 CP, ΔC100 CP, Chloramphenicol acetyl transferase protein, molecular weight marker).

### DETAILED DESCRIPTION OF THE INVENTION

The invention describes isolated Beak and feather disease virus nucleotide sequences having at least 70%, preferably at least 80%, more preferably at least 90% and even more preferably at least 95% sequence identity to a sequence set out in any one of SEQ ID NOS: 1 to 4, or a fragment thereof which encodes an amino acid sequence that is capable of generating an immunogenic response directed against a circovirus. The circovirus may be a Beak and Feather disease virus or a related virus, such as Pigeon circovirus, Duck circovirus, Goose circovirus or Columbid circovirus.

Also provided by the invention are nucleic acids that specifically hybridize to the nucleic acids herein disclosed under sufficient stringency conditions to selectively hybridize to the disclosed nucleic acids. Thus, nucleic acids for use, for example, as vaccines or as primers and probes to detect or amplify the target nucleic acids are contemplated herein. Specific or selective hybridization is that hybridization wherein the nucleic acid binds the target nucleic acid with minimal background, nonspecific hybridization to non-target nucleic acids. Typically, the stringency of hybridization to achieve selective hybridization is about 5°C to 20°C below the Tm (the melting temperature at which half of the molecules dissociate from its partner), but it is further defined by the salt concentration and the permitivity of the solution. Hybridization temperatures are typically higher for DNA-RNA and RNA-RNA hybridizations. The washing temperatures can similarly be used to achieve selective stringency, as is known in the art (Sambrook et al., 1987).

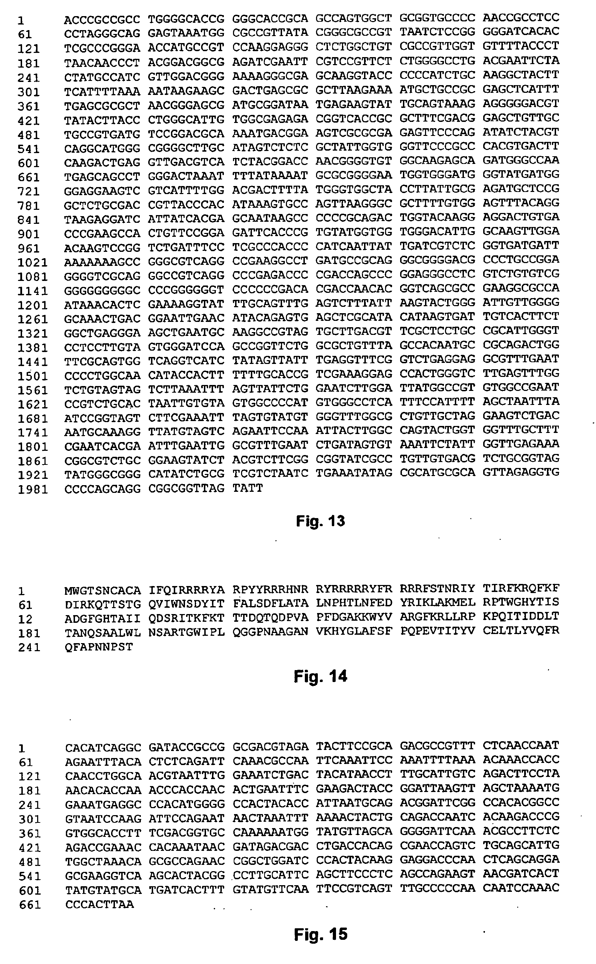

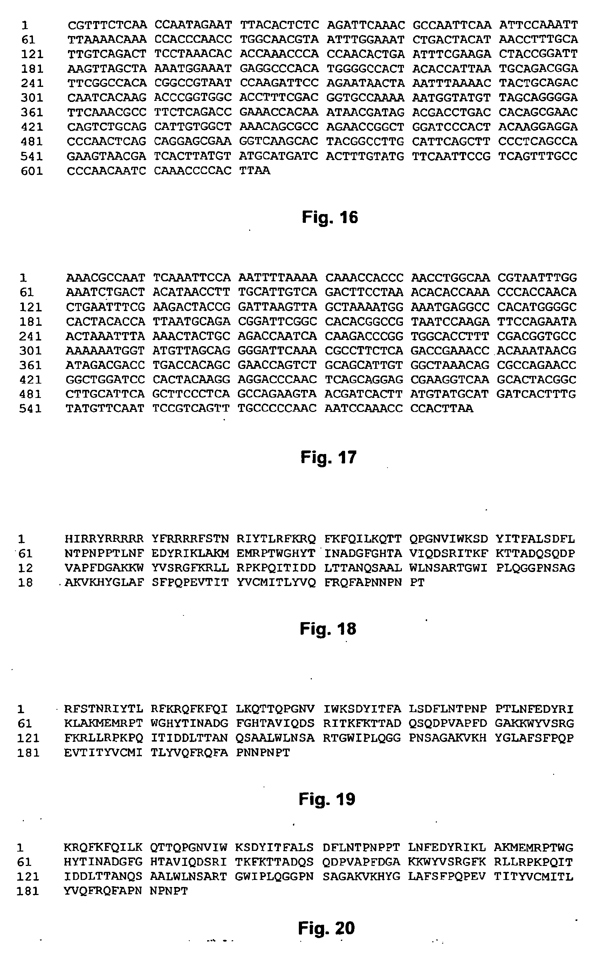

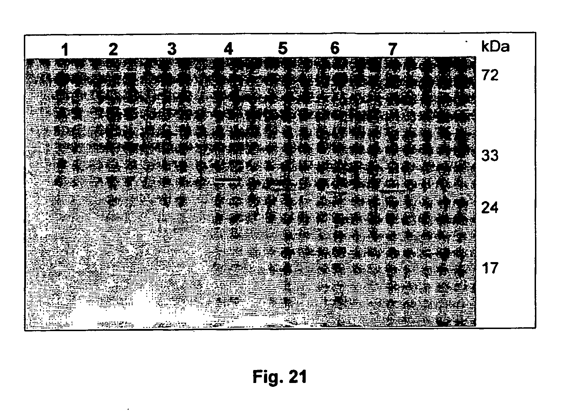

A Beak feather disease virus (BFDV) vaccine can be produced from these sequences, the vaccine including a protein encoded by the sequences, such as a BFDV virus coat protein (CP) having at least 70%, preferably at least 80%, more preferably at least 90% and even more preferably at least 95% sequence identity to a sequence set out in any one of SEQ ID NOS: 5 to 8, or an immunogenic protein fragment produced in an appropriate cell culture system. For example, the fragment may be a variant of a protein sequence which includes one or more truncations, substitutions, deletions or insertions. These variations are typically made so as to either enhance the efficacy of the protein in stimulating an immune response or render it safer for use in an animal. Similarly, the nucleotide sequences may be codon-optimised or otherwise modified to make production of the polypeptides inducible or repressible, for example, depending on the expression system used. Examples of protein fragments are shown in SEQ ID NOS: 19 to 25 (Figures 13 to 20) and Figure 21. The vaccine composition may contain a pharmaceutically acceptable carrier and/or adjuvant. Suitable carriers and adjuvants are known in the art.

Antibodies produced in an animal vaccinated with the vaccine can be isolated and used as a vaccine and/or for assay purposes.

The invention also discloses novel primers having nucleotide sequences set out in SEQ ID NOS: 9 to 17. These could be produced on a commercial scale for diagnostic purposes.

Pseudovirions can also be used in the invention as recombinant vaccine products. These comprise BFDV coat protein produced from a DNA construct in cell culture encapsidating single-stranded circular (ssc) virus-derived recombinant nucleotide sequence made in the same cells by the agency of the viral Rep (replication-associated protein), expressed in *cis* or in *trans*: this sscDNA could contain the virus coat protein gene and replication origin and cis-acting sequences, with *Rep* replaced with a marker gene, to provide a non-replicating coat protein gene DNA vaccine. Alternatively, the coat protein gene could also be replaced with another vaccine gene, selected from those known in the art. In another application, the nucleotide sequence could constitute the whole BFDV genome, or the native genome with the coat protein gene replaced: in the first instance, the nucleotide sequence would reconstitute presumably infectious virions, whose infectivity could be tested in live birds and which could be inactivated using standard techniques for use as a vaccine; in the second, a combination replicative DNA vaccine expressing a range of desirable vaccine proteins could be made, encapsidated in BFDV coat protein for easy delivery. Delivery of the pseudovirions or inactivated virions could be parenteral or oral.

Virions made in culture, or preferably, pseudovirions carrying a marker gene - such as green fluorescent protein (GFP) - could also be used for assaying the neutralising effects of antisera. In the first instance, standard tissue culture infectivity assays or LD/ID₅₀ animal assays could be used to assay antiserum potency. In the latter case, the expression of the marker gene in tissue cultured cells or in inoculated animals could be assayed after mixing the pseudovirions with immune serum.

Polyclonal avian antibodies could also be used as a potential therapeutic treatment for PBFD. Serum collected from heavily infected birds could be used to treat other birds during the less advanced stages of the disease. The use of infectious serum is, however, not recommended since it could subsequently have devastating affects on the general health of breading stocks.

A safer alternative to this practice would be to produce purified antibodies to BFDV by immunising laying hens with the purified BFDV protein or fragment. These hens could be kept for the sole purpose of obtaining large quantities of antibodies. A single hen's egg may yield up to 250 mg of IgY, making this system especially suitable for the maintenance of a critical mass of antibody stocks throughout the year (Schade *et al.,* 2001). This would be a critical factor in the successful implementation commercial venture should this approach prove to be effective. The protocol that is described in the examples below is a generalized method used to purify antibodies from eggs (Polson *et al*. 1990), and it will be understood that this protocol could be suitably modified or adapted, or alternatively, a different protocol could be used.

Apart from producing antibodies in chickens, it is envisaged that the antibodies could also be derived from rabbits.

The invention further describes a BFDV antibody detection assay to be used to determine whether or not birds have been vaccinated, and/or to detect past BFDV infections. It is envisaged that such an assay would be suitable for commercial use. The assay comprises antibodies derived as described herein from chickens or rabbits above and the cloned proteins or fragments thereof. This assay is much less cumbersome, considerably cheaper and more sensitive than the haemagglutination test that forms part of the prior art.

Vaccine compositions can be administered to a subject or an animal model by any of many standard means for administering the particular composition. For example, compositions can be administered orally, sublingually, intraocularly, intranasally, intravenously, by intramuscular injection, intradermally, by intraperitoneal injection, parentally, topically, transdermally or the like. Compositions can be administered alone or in combinations, for example, as a complex with cationic liposomes, encapsulated in anionic liposomes, enclosed in chochleates, or they can be encapsulated in microcapsules. Compositions can include various amounts of the selected composition in combination with a pharmaceutically acceptable carrier and, in addition, if desired, may include other medicinal agents, pharmaceutical agents, carriers, adjuvants, diluents, and the like. Injectables can be prepared in conventional forms, either as liquid solutions or suspensions, solid forms suitable for solution or suspension in liquid prior to injection, or as emulsions.

The present invention is further described by the following examples. Such examples, however, are not to be construed as limiting in any way either the spirit or scope of the invention.

### Examples:

### PBFD genome sequences

Heparinised blood samples were collected from 22 birds showing clinical signs of beak and feather disease. The full-length BFDV genome was amplified by inverse polymerase chain reaction (PCR) from individual blood samples using the primers *BFDV BamHi forw* 5' GGATCCA(G/T)CCGGTTCTGGC(G/A) 3' (SEQ ID NO: 9) and *BFDV BamHI rev* 5' GGATCCCACTACAAAGGAGGACCC 3' (SEQ ID NO: 10). The PCR was carried out at 94°C for 30s, 55°C for 30s and 68°C for 3min for 25 cycles using Expand Long template PCR System (Roche Diagnostics) as prescribed by the manufacturer.

The resulting PCR products (approximately 2Kb) were cloned into the pGEM-T-Easy plasmid (Promega) for sequencing. The clones were end sequenced with commercially available M13 primers (Perkin-Elmer) using the ABI Big Dye Terminator cycle sequencing kit on the Aplied Biosystem 3738 A DNA sequncing system. Based on previously published sequnce data (Basammi et al, 2001), three additional primers *BFDV I for1* 5' GTATCGCCTGATGTGACGTCTG 3' (SEQ ID NO: 11), *BFDV I for2* 5' CTGGACATTGTGGCGAGAGAC 3' (SEQ ID NO: 12) and *BFDV I for3* 5' GACCGTTACCACCATAAAGTG 3' (SEQ ID NO: 13) were designed and used to sequence the remainder of genome.

Four full-length BFDV genome nucleotide sequences, BFDV AfrG3-RSA (SEQ ID NO: 1), BFDV AfrG4-Gp (SEQ ID NO: 2), BFDV UC2-WC (SEQ ID NO: 3), and BFDV BCL1-ZAM (isolated from blood of a Black-cheeked Lovebird from Zambia) (SEQ ID NO: 4) were edited and assembled using DNAman 5.2.9 (Lynnon BioSoft). Multiple sequence alignment and phylogenetic analysis of the full-length genome was done using the programs DNAman 5.2.9 and MEGA 2.1 (Kumar *et al.,* 2001).

### Expression of recombinant BFDV coat protein in Escherichia coli

The open reading frame encoding the putative coat protein of BFDV was identified based on previously published data and amplified from heparinised blood samples by PCR using primers *PBDF CP for* 5' CTGTGGGGCACCTCTAACTGC 3' (SEQ ID NO: 14) and *BFDV CP rev* 5' GTCTTTATTAAGTAACTGGGATTGTTAGGGGC 3' (SEQ ID NO: 15). The PCR was carried out at 94°C for 30s, 58°C for 30s and 72°C for 1 min for 25 cycles using Super-Therm DNA Polymerase (Southem Cross Biotechnologies) as prescribed by the manufacturer. The resulting PCR products (approximately 0.76 kb) of 12 isolates were cloned into the pGEM-T-Easy plasmid (Promega).

The clones were sequenced with commercially available M13 primers (Perkin-Elmer) using the ABI Big Dye Terminator cycle sequencing kit on the Applied Biosystem 3738 A DNA sequencing system. The isolate BFDV AfrG4-GP, representative of the viruses found in southern Africa, was selected for further research.

The coat protein gene was excised from the pGEM-T-Easy vector by restriction enzyme digestion with *Notl* and *Xhol*. and ligated into linearised pProEX™ HT prokaryotic expression vector (Gibco BRL). Recombinant clones were identified by restriction enzyme digestion analysis and the authenticity of the PBFD gene verified by automated sequencing using the commercial M13 primers.

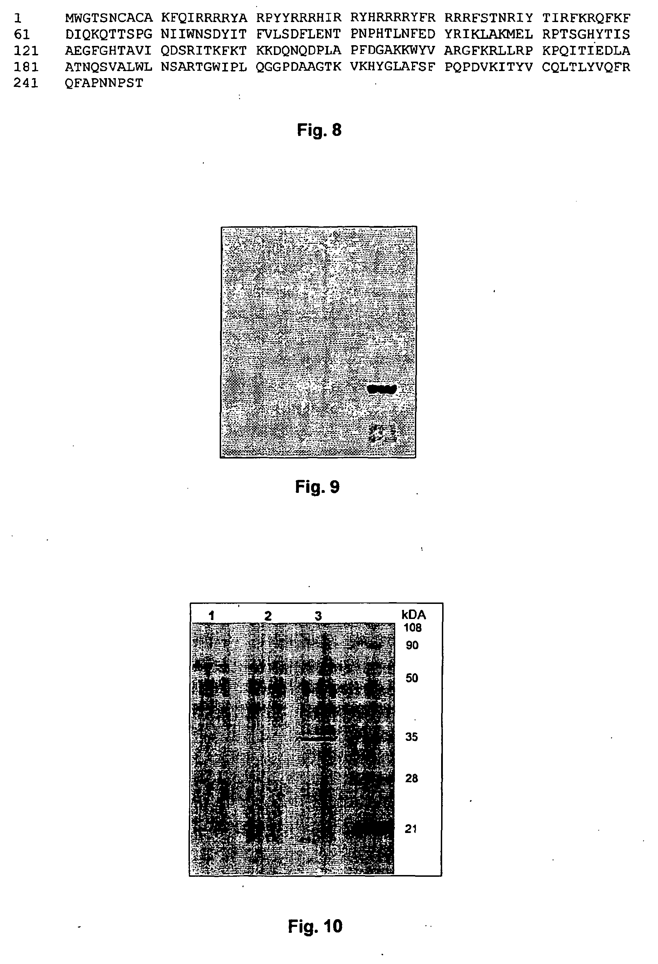

Expression of recombinant protein was induced by incubating cell cultures in the presence of 100 µg/ml ampicillin and 0.6 mM IPTG. Coat protein was detected by Western blot analysis (Figure 9). Cell lysates suspended in disruption buffer were boiled for 10 minutes and the proteins separated on a 12% denaturing SDS polyacrylamide gel. Separated polyproteins were transferred onto a nylon membrane (Osmonics) using a Transblot SD semi-dry transfer cell (BioRad). The membrane was subsequently probed with a α-His monoclonal antibody (Qiagen). Bound antibodies were detected with alkaline phosphatase-conjugated α-mouse antibodies.

Recombinant coat protein was purified from cell culture according to the manufacturer's instructions (Gibco BRL). Cells were lysed in Tris buffer containing 0.5 mM β-mercaptoethanol, 0.05 mM PMFS and 0.5 mg/ml lysozyme and incubated on ice for 30 minutes. Triton-X 100 was added to a final concentration of 1% and the lysate incubated at 37°C for 30 minutes. Contaminating DNA and RNA was removed by the addition of 10 ng/ml DNAse I and 0.1 mg/ml RNAse. Insoluble proteins were pelleted by centrifugation (14 000 x g for 15 minutes) and successively washed with phosphate buffer saline (PBS). The purified protein was resuspended in PBS.

### Expression of recombinant BFDV coat protein in insect cells

The coat protein gene was excised from the pGEM-T-Easy vector by restriction enzyme digestion with *EcoRI* and *XhoI* and ligated into linearised Bac-to-Bac baculovirus expression vector (Gibco BRL). Recombinant baculovirus carrying the BFDV coat protein gene was constructed according to the manufacturer's instructions.

Sf9 insect cells were infected with the recombinant baculovirus and recombinant coat protein detected by Western blot analysis (Figure 10). Cell lysates suspended in disruption buffer were boiled for 10 minutes and the proteins separated on a 12% denaturing SDS polyacrylamide gel. Separated polyproteins were transferred onto a nylon membrane (Osmonics) using a Transblot SD semi-dry transfer cell (Biorad). The membrane was subsequently probed with serum collected from birds showing clinical signs of beak and feather disease followed by polyclonal α-chicken serum. Bound antibodies were detected with alkaline phophatase-conjugated α-rabbit antibodies. Recombinant coat protein was purified from infected Sf9 cells. Cells were lysed in 20mM Tris-HCl buffer containing 1M KCI, 20 mM imidazole, and 0.1% Nonidet P-40 (v/v). Contaminating DNA and RNA was removed by the addition of 10 ng/ml DNAse I and 0.1 mg/ml RNAse. Soluble BFDV coat protein was purified by affinity chromatography using Ni-NTA agarose according to manufacturer's instructions (Qiagen). Fractions collected during the purification procedure were analysed SDS polyacrylamide gel electrophoresis.

### Replication of BFDV DNA in insect cells

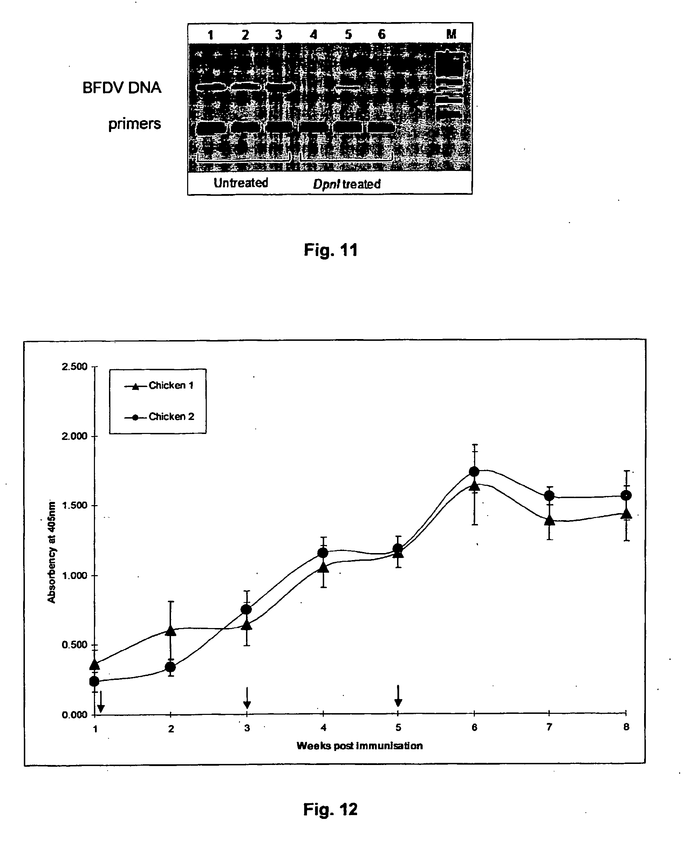

The ability of the Beak and feather disease virus genomic DNA to replicate in *Spodoptera frugiperda* cells (Sf21) was assayed by means of polymerase chain reaction (PCR) amplification. The BFDV genome was amplified from infected tissue and cloned into pGEM-T-Easy vector system. The genome was excised from the vector and circularized by ligation to produce a monomer, a partial dimer and a full dimer of the genome. 0.5 µg of each construct was transfected into *Spodoptera frugiperda* cells (Sf21). Transfection of the DNA was carried out with commercially available Lipofectamine. Total DNA was extracted from the transfected cells 48 hours post-transfection. Samples were serially diluted and the input plasmid-derived viral DNA was removed by *DpnI* endonuclease restriction enzyme digest, which cleaves only input bacterial (=methylated) DNA. DNA samples were subsequently analysed for the presence of replicative forms of the BFDV genome by PCR using primers 5' AGATCTATGCCGTCCAAGGAGGGATCTG 3' (SEQ ID NO: 16) and 5' AAGCTTCTAATAATTGATGGGGTGGGCGAG 3' (SEQ ID NO: 17) (sequences engineered to create endonuclease restriction sites are indicated in bold type) designed to amplify the BFDV Rep gene (Figure 11).

### Immunogenicity of BFDV CP protein produced in insect cells

Twenty four week-old White Leghorn chickens were immunised intramuscularly with 50 pg purified BFDV capsid protein obtained above, emulsified in Freund's incomplete adjuvant. The birds were boosted 14 and 21 days post-immunisation (p.im) with the same amount of the respective antigens.

Eggs laid by the immunised chickens were collected at regular intervals and stored at 4 °C. All eggs were processed within 7 days of being laid. Immunoglobulin Y (IgY) was extracted from the eggs as described previously (Polson, 1990). Briefly, egg yolks were separated from the egg white and washed with dd.H₂O. The yolk sacks were punctured and two volumes of phosphate buffer (100 mM NaH₂PO₄, pH 7.4) were added to the yolk. Crushed PEG 6000 was added to 3.5% (w/v) and dissolved by rotary inversion. The vitelin fraction was pelleted by centrifugation (5000 x g, 10 minutes) and contaminating lipids removed by filtering the supernatant though cotton wool. The concentration of PEG was adjusted to 12% by adding 8.5 % (w/v) PEG 6000 to the filtrate. The solution was mixed, centrifuged (12 000 x g, 10 minutes), and the pellet dissolved in phosphate buffer (equal volume to that obtained after filtration). The IgY was again precipitated with 12% (w/v) PEG 6000, before dissolving it in 1/10^{th} of the original egg yolk volume using phosphate buffer.

The reactivity of the purified IgY to the recombinant protein was assessed by ELISA. Polysorb 96-well microtitre plates were coated with crude lysate of insect cells infected with recombinant baculovirus expressing BFDV CP. The target antigen was diluted in carbonate buffer (50 mM N₂CO₃, pH9). The wells were block for 1h at room temperature with 5% skimmed milk powder dissolved in PBS-Tween buffer. Serial dilutions of pooled sera samples were added to the wells and incubated for 3h at room temperature. The plates were washed x 3 PBS-Tween buffer after which rabbit anti-chicken polyclonal antibodies were added to each well. Bound antibodies were detected with alkaline-phophatase conjugated goat anti-mouse monoclonal antibodies using p-nftrophenyl phosphate (Sigma) as substrate. Plates were read at 405 nm on a PowerWave™ XS universal microplate Spectrophotometer.

The results are shown in Figure 12. Chickens sero-converted 2 weeks post immunization. The level of the response was dose dependent with an increase in the average antibody titres following boosting with purified BFDV CP. Antibody titres remained high for the duration of the trial.

While the invention has been described in detail with respect to specific embodiments thereof, it will be apparent to those skilled in the art that various alterations, modifications and other changes may be made to the invention without departing from the spirit and scope of the present invention. It is therefore intended that the scope of the invention covers or encompasses all such modifications, alterations and/or changes.

### References

Bassami, M.R., Berryman, D., Wilcox, G.E. & Raidal, S.R. (1998) Psittacine beak and feather disease virus nucleotide sequence analysis and its relationship to porcine circovirus, plant circovirus and chicken anemia virus. Virology. 249, 453-459.
Bassami, M.R., Ypelar, I., Berryman, D., Wilcox, G.E. & Raidal, S.R. (2001). Genetic Diversity of beak and feather disease virus detected in psittacine species in Australia. Virology. 276, 392-400*.*
Greenacre, C.B., Latimer, K.S., Niagro F.D. (1992) Psittacine beak and feather disease virus infection in scarlet macaw (Amazona macao). Journal of the Association of Avian Veterinarians. 2, 95-98.
Latimer, K.S., Rakich, p.m., stiffens, W.L, Kircher, I.M., Richie, B.W., Niagro, F.D. & Lukert, P.D. (1991). A novel DNA virus associated with feather inclusions in psittacine beak and feather disease. Veterinary Pathology. 28, 300-304.
McOrist, S. (1998). Some diseases of free-living Australian Birds. ICBP Technical Publication. 16, 13-38.
Meehan, B.M., Creelan, J.L., McNulty, M.S. & Todd, D. (1997). Sequence of porcine circovirus DNA: affinities with plant circoviruses. Journal of General Virology. 78, 221-227.
Meehan, B.M., McNeilly, F., Todd, D. Kennedy, S., Jewhurst, V.A., Ellis, J.A., Hassard, L.E., Clark, E.G., Haines, D.M. & Allan, G.A. (1998). Characterisation of novel circovius DNAs associated wasting syndromes in pigs. Journal of General Virology. 79, 2171-2179.
Niagro, F.D., Forsthoefel, A.N., Lawther, R.P., Kamalanathan, L., Ritchie, B.W., Latimer, K.S. & Lukert, P.D. (1998). Beak and feather disease virus and porcine circovirus genomes: Intermediates between the geminiviruses and plant circoviruses. Archives of Virology. 143, 1723-1744.
Noteborn, M.H., de Boer, G.F., van Roozelaar, D.J., Karreman, C., Kranenburg, O., Vos, J.G., Jeurissen, S.H., Hoeben, R.C., Zanteman, A. & Koch, G. (1991). Characterisation of cloned chicken amemia virus DNA that contains elements for the infectious replication cycle. Journal of Virology. 65, 3131-3139.
Pass, D.A. & Perry, R.A. (1984). The pathology of psittacine beak and feather disease. Australian Veterinary Journal. 61, 69-74.
Phenix, K.V., Weston, J.H., Ypelaar, I., Lavazza, A., Smyth, J. A., Todd, D., Wilcox., G.E. & Raidal, S.R. (2001). Nucleotide sequence analysis of a novel circovirus of canaries and its relationship to other members of the genus Circovirus and of the family Circoviridae. Journal of General Virology. 82, 2805-2508.
Polson, A., Coetzer, T., Kryger, J., van Mltzahn, E. & van den Merwe, K.J. (1985). Improvement in the isolation of IgY from the yolks of eggs laid by immunized hens. Immunological Investigations. 14, 323-327.
Polson A. (1990). Isolation of IgY from the yolks of eggs by a chloroform polyethylene glycol procedure. Immunological Investigations. 19(3), 253-258.
Raidal, S.R., Firth, G.A. & Cross, G.M. (1993). Vaccination and challenge studies of psittacine beak and feather disease virus. Australian Veterinary Journal. 70, 437-441.
Raidal, S.R., McElnea, C.L. & Cross, G.M. (1993) Seroprevalence of psittacine beak and feather disease viru in wild psittacine birds in New South Wales. Australian Veterinary Journal. 70, 137-139.
Raidal, S.R., Sabine, M. & Cross, G.M. (1993). Laboratory diagnosis of psittacine beak and feather disease by haemagglutination and haemagglutination inhibition. Australian Veterinary Journal. 70, 133-7.
Ritchie, B.W. & Carter, K. (1995). Avian Viruses: Function and control. Wingers Publishing. Florida, USA.
Ritchie, B.W., Niagro, F.D., Latimer, K..S., Steffens, W.L., Pesti, D., Campagnoli. R.P. & Lukert P.D. (1992). Antibody response to and maternal immunity from a experimental psittacine beak and feather disease vaccine. American Journal of Veterinary Research. 9, 1512-4518.
Ritchie, B.W., Niagro, F.D., Latimer, K.S., Lukert P.D., Steffen, W.L., Rikich, P.M. & Pritchart, N. (1990). Ultrastructural, proteincomposition, and antigenic comparison of psittacine beak and feather disease virus purified from four genera of psittacine birds. Journal of Wildlife Disease. 26, 196-203.
Ritchie, B.W., Niagro, F.D., Latimer, K.S., Steffens, W.L., Pesti, D. & Lukert, P.D. (1991). Hemagglutination by psittacine beak and feather disease virus and use of hemagglutination inhibition for detection of antibodies against the virus. American Journal Veterinary Research. 52, 1810-5.
Ritchie, B.W., Niagro, F.D., Lukert, P.D., Steffens, I.W.L. & Latimer, K.S. (1989). Characterisation of a new virus from cockatoos with psittacine beak and feather disease. Virology. 171, 83-88.
Sambrook et al., Molecular Cloning. A Laboratory Manual, 2nd Ed., Cold Spring Harbor Laboratory, Cold Spring Harbor, N.Y., 1989; Kunkel et al. Methods Enzymol. 1987:154:367, 1987.
Sanada, N. & Sanada, Y. (2000). The sensitivities of various erythrocytes in a haemagglutination assay for the detection of psittacine beak and feather disease virus. Journal of Veterinary Medicine and Infectious Disease. 47 6, 441-3.
Schade, R., Behn, I., Erhard, M., Hlinak, A. & Staak, C. (2001). Chicken Egg Yolk Antibodies, Production and Application, IgY-Technology. Springer-Verlag Berlin Heidelberg Publishing. New York, USA.
Todd, D. (2000). Circoviruses: immunosuppressive threat to avian species: a review. Avian Pathology 29, 373-394.
Woods, L.W. & Latimer, K.S. (2000). Circovirus infections of nonpsittacine birds. Journal of Avian Medicine and Surgery. 14, 154-163.
Wylie, S.L. & Pass, D.A. (1987). Avian Pathology. 19, 269-274.
Ypelaar, I., Bassami., M.R., Wilcox, G.E. & Raidal, S.R. (1999). A universal polymerase chain reaction for the detection of psittacine beak and feather disease virus. Veterinary Microbiology. 68, 141-8.

## Claims

1. A composition comprising a beak and feather disease virus (BFDV) coat protein polypeptide:
(a) having an amino acid sequence which has at least 80% identity to a sequence set out in any one of Figures 5 to 8 which is capable of generating an immunogenic response directed against a circovirus and wherein in said composition said protein encapsidates a circovirus-derived single stranded circular nucleotide sequence containing replication origin and cis-acting sequences necessary for replication, wherein:
(b) the encapsidated single-stranded circular nucleotide sequence is replicated by BFDV Rep expressed in trans;
(c) the encapsidated single-stranded circular nucleotide sequence is a native BFDV genome, and constitutes an infectious virion when encapsidated by the BFDV coat protein;
(d) the encapsidated single-stranded circular nucleotide sequence is a BFDV genome with its Rep gene replaced, and is non-replicating in transfected cells or in birds; or
(e) the encapsidated single-stranded circular nucleotide sequence is a BFDV genome with its coat protein gene replaced with another gene, and is replication-competent in transfected cells or in birds;
for use in a method of preventing and/or treating a circovirus infection.

2. The composition according to Claim 1, wherein the circovirus is beak and feather disease virus.

3. The composition according to Claim 1 or Claim 2, wherein the circovirus-derived single-stranded circular nucleotide sequence is a BFDV sequence having at least 95% identity to a sequence as set out in any one of Figures 1 to 4.

4. The composition according to any one of Claims 1 to 3, wherein the amino acid sequence has at least 95% identity to a sequence set out in any one of Figures 5 to 8.

5. A vaccine comprising a composition as claimed in any one of Claims 1 to 4 for use in preventing and/or treating a circovirus infection.

6. A recombinant vaccine for BFDV comprising a pseudovirion comprising a BFDV polypeptide having an amino acid sequence set out in any one of Figures 5 to 8 produced from a DNA construct in cell culture encapsidating single-stranded circular (ssc) virus-derived recombinant nucleotide sequence made in the same cells by the agency of the viral Rep (replication-associated protein), express in *cis* or in *trans.*

7. An assay for quantification of serum antibody immune response in vaccinated birds, which assay includes the composition of any one of Claims 1 to 4, or infectious virions formed thereby.

## Patentansprüche

1. Zusammensetzung, die ein Hüllprotein-Polypeptid des Schnabel- und Federkrankheit-Virus (BFDV) enthält,
a) das eine Aminosäuresequenz aufweist, die eine mindestens achtzigprozentige Identität mit einer der in den Figuren 5 bis 8 festgelegten Sequenzen hat, die eine gegen ein Circovirus gerichtete Immunantwort erzeugen kann, und wobei dieses Protein in dieser Zusammensetzung eine vom Circovirus abgeleitete einsträngige zirkulare Nukleotidsequenz enkapsidiert, die einen Replikationsursprung und die für die Replikation notwendigen cis-wirksamen Sequenzen enthält, wobei
b) die enkapsidierte einsträngige zirkuläre Nukleotidsequenz durch trans-exprimiertes BFDV Rep repliziert wird,
c) die enkapsidierte einsträngige zirkuläre Nukleotidsequenz ein natives BFDV-Genom ist und ein infektiöses Virion darstellt, wenn sie mit dem BFDV-Hüllprotein enkapsidiert ist,
d) die enkapsidierte einsträngige zirkuläre Nukleotidsequenz ein BFDV-Genom mit ersetztem Rep-Gen ist und in transfizierten Zellen oder in Vögeln nicht repliziert, oder
e) die enkapsidierte einsträngige zirkuläre Nukleotidsequenz ein BDFV-Genom ist, dessen Hüllprotein durch ein anderes Gen ersetzt wurde und das in transfizierten Zellen oder in Vögeln replikationsfähig ist,
zur Verwendung in seinem Verfahren zur Verhütung und/oder Behandlung einer Circovirus-Infektion.

2. Zusammensetzung nach Anspruch 1, wobei das Circovirus das Schnabel- und Federkrankheit-Virus ist.

3. Zusammensetzung nach Anspruch 1 oder 2, wobei die von einem Circovirus abgeleitete einsträngige zirkuläre Nukleotidsequenz eine BFDV-Sequenz mit mindestens 95 % Identität mit einer der in den Figuren 1 bis 4 dargestellten Sequenzen ist.

4. Zusammensetzung nach einem der Ansprüche 1 bis 3, wobei die Aminosäuresequenz mindestens 95 % Identität mit einer der in den Figuren 5 bis 8 dargestellten Sequenzen hat.

5. Impfstoff, der eine Zusammensetzung nach einem der Ansprüche 1 bis 4 enthält, zur Verwendung bei der Verhütung und/oder Behandlung einer Circovirus-Infektion.

6. Rekombinanter Impfstoff für BFDV, der ein Pseudovirion mit einem BFDV-Polypeptid umfasst, das eine der in den Figuren 5 bis 8 dargestellten Aminosäuresequenzen, in Zellkultur aus einem DNA-Konstrukt hergestellt, aufweist, das eine einsträngige zirkuläre (ssc), von einem Virus abgeleitete rekombinante Nukleotidsequenz enkapsidiert, die in den gleichen Zellen durch Vermittlung des in cis oder trans exprimierten viralen Rep (replikationsverbundenes Protein) hergestellt wurde.

7. Assay zur Bestimmung der serum-Antikörperimmunreaktion in geimpften vögeln, der die Zusammensetzung nach einem der Anspräche 1 bis 4 oder **dadurch** gebildete infektiöse Virionen umfasst.

## Revendications

1. Composition comprenant un polypeptide de protéine de coque du virus de la maladie du bec et des plumes (BFDV) :
(a) possédant une séquence d'acides aminés ayant au moins 80 % d'identité avec une séquence présentée sur l'une quelconque des figures 5 à 8 qui est capable de générer une réponse immunitaire dirigée contre un circovirus et ladite protéine encapsidant dans ladite composition une séquence de nucléotides circulaires simple brin dérivée d'un circovirus et contenant l'origine de réplication et des séquences à action *cis* nécessaire pour la réplication, dans laquelle :
(b) la séquence de nucléotides circulaires simple brin encapsidée est répliquée par la protéide du BFDV. Rer exprimée en *trans* ;
(c) la séquence de nucléotides circulaires simple brin encapsidée est un génome natif du BFDV, et constitue un élément infectieux lorsqu'il est encapsidée par la protéine de coque du BFDV ;
(d) la séquence de nucléotides circulaires simple brin encapsidée est un génome BFDV ayant son gêne Rep remplacé et ne se réplique pas dans des cellules transfectées ou chez les oiseaux ; ou
(e) la séquence de nucléotides circulaires simple brin encapsidée est un génome du BFDV ayant on gène de protéine de coque remplacé par un autre gène, et est de réplication dans des cellules transfectées ou chez des oiseaux ;
pour une utilisation dans un procédé de prévention de traitement d'une par circovirus.

2. Composition selon la revendication 1, dans laquelle le circovirus et un virus de la maladie du bec et des plumes.

3. Composition selon la revendications 1 ou 2, dans laquelle la séquence due nucléotides circulaires simple brin dérivée d'un circovirus est une séquence du BFDV. possédant au moins 95 % d'identité avec une séquence telle que présentée sur l'une quelconque des figures 1 à 4.

4. Composition selon l'une quelconque des revendications 1 à 3, dans laquelle la séquence d'acides aminés possède au moins 95 % d'identité avec une séquence présentée sur l'une quelconque des figures 5 à 8.

5. Vaccin comprenant une composition selon l'une quelconque des revendications 1 à 4, pour une utilisation dans la prévention et/ou le traitement d'une infection par circovirus.

6. Vaccin recombinant pour le BFDV. comprenant un pseudovirion comprenant un polypeptide du BFDV possédant une séquence d'acides aminés présentée sur l'une quelconque des figures 5 à 8, produit à partir d'une construction d'ADN dans une culture de cellules encapsidant une séquence de nucléotides recombinante dérivée d'un virus circulaire simple brin (ssc) dans les mêmes cellules par l'agencement de la protéine Rep (protéine associée à la réplication) virale, exprimée en *cis* ou sen *trans.*

7. Analyse de d'une réponse immunitaire à un anticorps sérique chez des oiseaux vaccinés laquelle analyse comprend la composition selon l'une quelconque des revendications 1 à 4, ou des virions infectieux formés à partir de celle-ci.
